# EUROPEAN PATENT SPECIFICATION

(11) **EP 4 117 771 B1**
(45) Date of publication and mention of the grant of the patent: **11.11.2026**
(21) Application number: 21768873.8
(22) Date of filing: 08.03.2021
(51) Int. Cl.: A61N 2/02, A61N 2/06

(54) **PULSED ELECTROMAGNETIC FIELD DEVICE WITH SUSTAINED MODULATION**
VORRICHTUNG FÜR GEPULSTE ELEKTROMAGNETISCHE FELDER MIT KONTINUIERLICHER MODULATION
DISPOSITIF À CHAMP ÉLECTROMAGNÉTIQUE PULSÉ À MODULATION CONTINUE

(30) Priority: 08.03.2020 US 202062986758 P
(43) Date of publication of application: 18.01.2023
(73) Proprietor: Clineticz, LLC, Georgetown, TX 78628 (US)
(72) Inventor: CLICK, George Michael, Georgetown, TX 78628 (US); YEAGER, Rick B., Austin, TX 78759 (US); IZZARD, Dan A., Georgetown, TX 78633 (US)
(74) Representative: Berggren Oy
(86) International application number: PCT/US2021/021295
(87) International publication number: WO 2021/183410

(56) References cited:
- WO-A1-91/04764
- KR-A- 20180 052 402
- KR-B1- 100 857 210
- US-A- 6 149 577
- US-A1- 2003 158 583
- US-A1- 2008 243 197
- US-A1- 2012 253 101
- US-A1- 2016 228 723
- US-A1- 2018 078 780
- US-A1- 2018 126 185
- US-A1- 2019 126 036
- US-A1- 2019 391 217

## Description

### FIELD OF INVENTION -

This patent application relates to pulsed electromagnetic field (PEMF) devices. More particularly, the application relates to PEMF devices where magnetic flux is created by one or more coil positioned in proximity to permanent magnets.

### BACKGROUND - Prior Art

Prior art PEMF devices generate electromagnetic field flux and induce voltage within the body by varying current through a coil. This non-invasive technique produces beneficial results as demonstrated in thousands of studies and research papers over more than five decades. The strength of the electromagnetic field can be increased by increasing the number of coil turns, by increasing current through a coil, or by increasing both the number of coils and the current. Magnetic field flux is designated as dB/dt where the "dB" represents the change in the magnetic field "B" for a time interval "dt".

Low to medium intensity PEMF devices can produce a peak magnetic field up to about 0.02 T (200 gauss) at high pulse frequencies directly from AC or battery power supplies. High intensity "impulse devices" such as transcranial magnetic stimulation (TMS) devices typically produce brief high amperage pulses at a peak intensity of 0.1-2.5+ T (1,000-25,000+ Gauss), once or twice per second, by charging and discharging capacitors.

A 2003 NASA study reported that fast rise-time pulses promoted a 4X improvement for *in vitro* stem cell growth, but that sinusoidal pulses were not effective. US Pat. No. 8,376,925 to Dennis et al describes commercial versions of high slew rate trapezoidal-wave pulsed electromagnetic field devices and the ICES-PEMF^{™}, a low to medium intensity PEMF device.

US Patent No. 10,500,408 to Helekar et al. proposes advantages of a rotating permanent magnet device over conventional TMS devices. Strong permanent magnets can provide a strong magnetic field, but the flux created by rotating magnets is sinusoidal and less that effective than fast rise time coil pulses.

Another PEMF device with a plurality of permanent magnets and a coil is described in US 6 149 577 A.

Most PEMF devices are operated at a frequency of 7-100 Hz. Some studies have reported that pathogen-specific "targeted frequencies" between about 1000 Hz and 2 MHz can kill or disable pathogens.

### SUMMARY

The invention is defined by the claims.

There are two fundamental limitations of coil-based PEMF devices to create effective magnetic field flux within tissue. First, the magnetic field strength drops sharply with distance from the coil with a decrease of 50% in the first 1.27 cm (0.5 inch) and over 99% in the first 7.62 cm (3 inches). Second, it is not practical for an affordable device to create a strong magnetic field with pulse frequencies greater than a few pulses per second.

Applicants suggest that prior art PEMF devices cannot deliver combinations of pulse intensity and pulse frequency that effectively address many ailments. Low or medium intensity devices appear to have insufficient pulse intensity, and high intensity impulse devices appear to be have insufficient pulse frequency, to address many chronic conditions.

A PERMAFLUX^{™} PEMF device has a flux module comprising a coil assembly having at least one coil configured in proximity to a permanent magnet assembly with at least one permanent magnet. The permanent magnets deliver a strong and persistent magnetic field deep into tissue. A coil controller employs pulse width modulation and a phase controller to deliver a series of fast-rise-time current pulses to a first coil to "ripple" the magnetic field.

The flux module can deliver a sustained modulation with duty cycles that are orders of magnitude higher than impulse PEMF devices, so PERMAFLUX can exceed the effective dosage of impulse devices by delivering many more pulses at a lower, and more benign, intensity. This ripple creates significant magnetic flux deep into the tissue without the need to recreate a strong magnetic field with each pulse. The pulse duration can be significantly longer than what is practical with many impulse devices.

When the first coil is energized with a positive current direction, the coil generates an electromagnetic field with the same polarity as the magnet assembly and thereby enhances the permanent magnetic field. When the first coil is energized with a negative current direction, the coil generates an electromagnetic field with the opposite polarity as the magnet assembly and thereby partially retracts the permanent magnetic field. A magnetic field flux is produced by the enhancement and retraction of the magnetic field. The device also appears to function as an antenna to transmit the coil electromagnetic field into adjacent tissue. Flux can be provided from a series of positive or negative pulses, an alternating series of positive and negative pulses, or other pulse patterns.

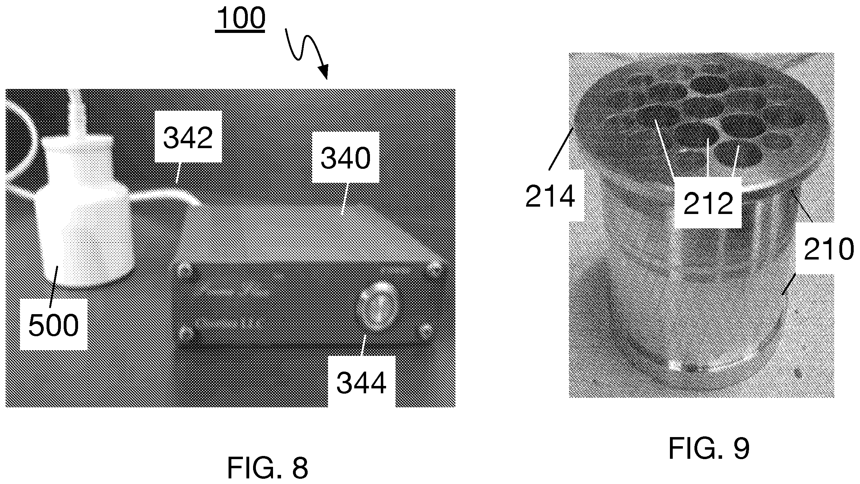

FIG. 8 is a front perspective view of an example AC powered PERMAFLUX device **100** showing an encapsulated flux module **500**, a control box **340** with a lighted on/off switch **344,** and cable **342** from the control box to the flux module **500**. In this example, power is provided from an AC outlet and converted to 12 volts DC with a transformer (not shown). Portable devices will provide the flux module, controls, and battery or capacitor power supply within a hand-held housing.

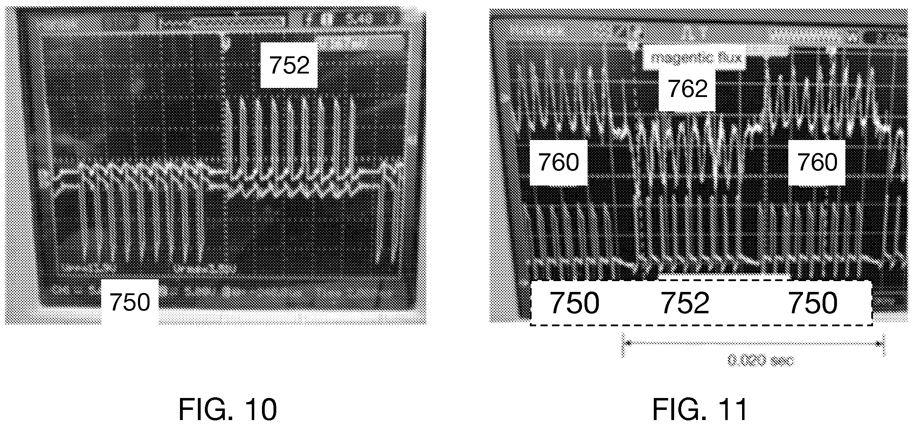

FIG. 10 is an oscilloscope display showing an example 50Hz coil signal with approximately 36% duty cycle. The lower trace shows a sequence **752** of nine sharp negative current pulses of about 300 microsecond duration which were generated by microprocessor pulse width modulation. The pulses have a rise time of less than 1 microsecond. The upper trace shows a subsequent sequence **750** of nine positive current pulses. In this example, a current of about 0.3 amps is directed to a 200-turn coil of 26 gauge copper wire wound around a permanent magnet assembly.

FIG. 11 shows an example upper trace analog display of the magnetic flux signal from a magnetic flux meter. The lower trace shows the positive current pulses **750,** and the negative current pulses **752.** This display is from a second oscilloscope and setup where the lower trace is displayed as inverted from FIG. 10. A magnetic field flux pulse is generated by each current pulse. The positive fluxes **760** created by the positive current pulses **750** are offset from the negative fluxes **762** created by the positive current pulses **752.** The flux pulses have a duration of approximately 1 millisecond and induce voltage in the brain or tissue.

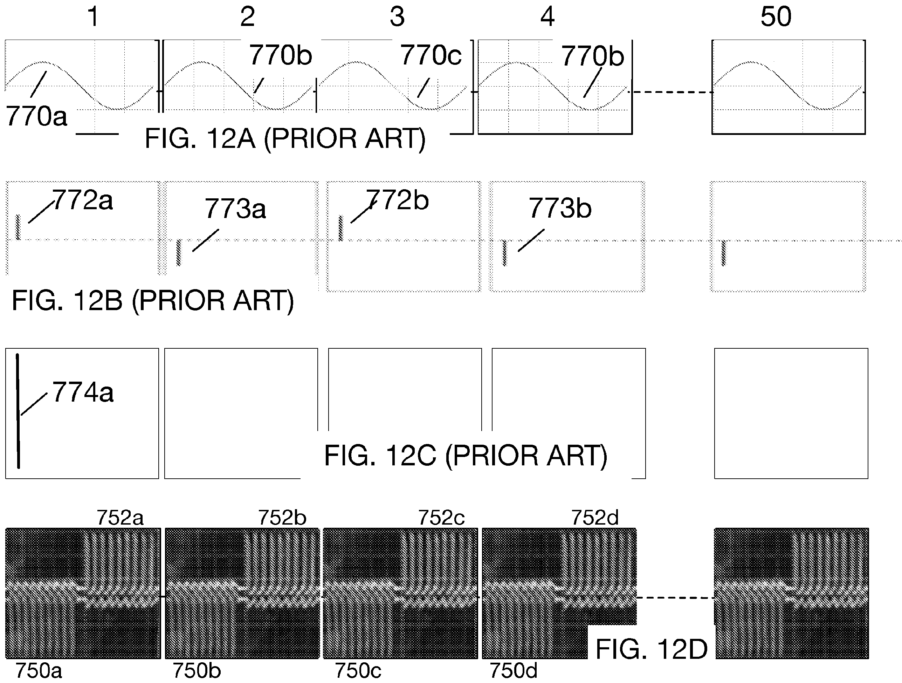

FIGs 12A-12D illustrate example pulse rates and relative intensities for prior art PEMF devices and PERMAFLUX over a one second time frame. For illustration, all devices are shown at the same 50Hz frame intervals 1-50.

FIG. 12A is an example sinusoidal resonant PEMF device with 50 sinusoidal pulses **770a, 770b, 770c, 770d** ... per second at a peak intensity of about 0.02 T (200 gauss) produced in each cycle. The slow change in coil current creates longer pulse rise times, resulting in a smaller dB/dt than the other devices.

FIG. 12B is an example PEMF device where sharp positive **772a, 772b,** ... or negative **773a, 773b,** ... current pulses of about 200 microsecond duration is delivered in each of the 50 cycles with a peak intensity of about 0. 02 T (200 gauss) produced in each cycle. Most of these devices are operated at a lower intensity. The magnetic field flux produced by this pattern is substantially stronger than in the sinusoidal devices because of the fast pulse rise times. The pulses are shown as vertical lines, but are actually trapezoidal in shape with steep rise and fall times.

FIG. 12C is an example of impulse PEMF devices where a single pulse with 3 nanosecond to 400 microsecond duration is delivered once per second. The example shows a single pulse **774a** in the first frame and no activity for frames 2-50. The magnetic field flux produced by this pattern is likely to be the strongest of the examples, if very short nanosecond pulses are not too fast to create effective flux, but the pulse rate is the slowest of the examples.

High intensity appears to be important, but not sufficient, to treat chronic ailments. If medium intensity resonant devices could be effective, then they should have been well-established in the last 15 years. Likewise, if impulse devices were effective, then more successes should have been documented in the last 10 years. Applicants suggest that both types of devices can offer some benefit, but that each has a fundamental limitation.

FIG. 12D is an example of PERMAFLUX operating at 50 Hz with nine negative current pulses **752a, 752b, 752c, 752d,** ... and nine positive current pulses **750a, 750b, 750c, 750d,** ... in each of the fifty 0.020 second cycles. This example delivers the highest pulse rate of 900 pulses per second, with each pulse having a fast rise time of less than 2 microseconds, and about 300 microsecond duration. In addition to the faster pulse rate, the magnetic field flux has a substantially deeper reach into tissue than a device with an equivalent coil size and current.

### REFERENCES

[1] The ARRL Antenna Book for Radio Communications, 24th Edition, Published by (ARRL) The American Radio Relay League, Inc.
[2] The ARRL Handbook for Radio Communications, (Six Volume Set), Ninety-Eighth Edition; First Printing (2020) Published by (ARRL) The American Radio Relay League, Inc.
[3] https://www.kjmagnetics.com/calculator.asp

### BRIEF DESCRIPTION OF FIGURES

FIG. 1 is a cross section view of an example device flux module.
FIG. 2 is a front view of the example flux module of FIG. 1.
FIG. 3 is a front view of a example magnet assembly with a plurality of cylindrical magnets.
FIG. 4 is a front perspective view of another magnet assembly.
FIG. 5 is an example of a flux module with two coils.
FIG. 6A is a cross section view of another example flux module.
FIG. 6B is a cross section view of another example flux module.
FIG. 6C is a cross section view of another example flux module.
FIG. 6D is a cross section view of another example flux module.
FIG. 6E is a cross section view of another example flux module.
FIG. 7 is an example pulse control schematic.
FIG. 8 is a front perspective view of a first embodiment device.
FIG. 9 is a top perspective view of an example magnet support.
FIG. 10 is an oscilloscope display below showing an example 50Hz coil signal.
FIG. 11is an oscilloscope display of magnetic flux pulses and coil pulses.
FIG. 12A (PRIOR ART) is an example 50 Hz sinusoidal PEMF device coil signal.
FIG. 12B (PRIOR ART) is an example a 50 Hz sharp pulse PEMF device coil signal.
FIG. 12C (PRIOR ART) is an example impulse PEMF with a 1 Hz coil signal.
FIG. 12D is an example embodiment 50 Hz coil signal with nine negative current pulses and nine positive current pulses each cycle.

### DESCRIPTION

In this specification, the term "PEMF" means pulsed electromagnetic field. The term "PEMF therapy" refers to the use of a PEMF device on a living organism, including the human body; dogs, horses, and other animals; or plants. The embodiments described herein can be considered PEMF devices where one or more coil supplies a pulsed electromagnetic field by "rippling" a permanent magnetic field, or PEMF devices where the combination of a permanent magnet assembly and a coil assembly acts as an antenna to broadcast the electromagnetic fields created by the coil and magnet assemblies.

The term "slew rate" refers to the calculated magnetic flux value "dB/dt" where dB is the change in magnetic field strength B, and "dt" is the change in time. Some PEMF devices with an ultra-fast pulse rise time can yield a high calculated dB/dt, but might have little practical benefit because of the small actual magnetic field intensity.

The term "coil controller" refers to the conversion of a power supply to a series of discrete current pulses to a coil. In some examples, the coil controller may be adjustable to produce various pulse intensities, pulse frequencies, and pulse durations.

"Pulse width modulation" is a method of controlling current to a device by producing trapezoidal waves, where input DC voltage is switched "on" and "off" to create nominal square waves. In this specification, the terms "current control" and "current controller" are used in preference to the terms "voltage control" or "voltage control".

The term "North polarity" means that a magnet assembly has magnet(s) with a North pole oriented toward a subject. The term "positive current" means the current direction through a coil which produces an electromagnetic field with North polarity oriented toward a subject.

Example embodiments describe a pulsed electromagnetic field device configured to apply a fluctuating electromagnetic field to a subject organism. The devices have at least one flux module comprising a permanent magnet assembly with at least one permanent magnet, and a coil assembly comprising at least one first configured in proximity to the permanent magnet assembly. A first coil controller is configured to intermittently apply current from a power supply to a first coil.

According to the invention the permanent magnet assembly comprises a plurality of parallel cylindrical magnets where the first coil is wound around the permanent magnet assembly and configured perpendicular to the longitudinal axes of the magnets.

The devices are held or supported near or against a subject organism, such as a human, animal, or plant. In one example, the pulse controller delivers a plurality of positive and negative current pulses with rise times less than 5 microseconds to generate magnetic filed flux and to induce voltage within the organism.

### Flux module with multiple cylindrical magnets and single coil

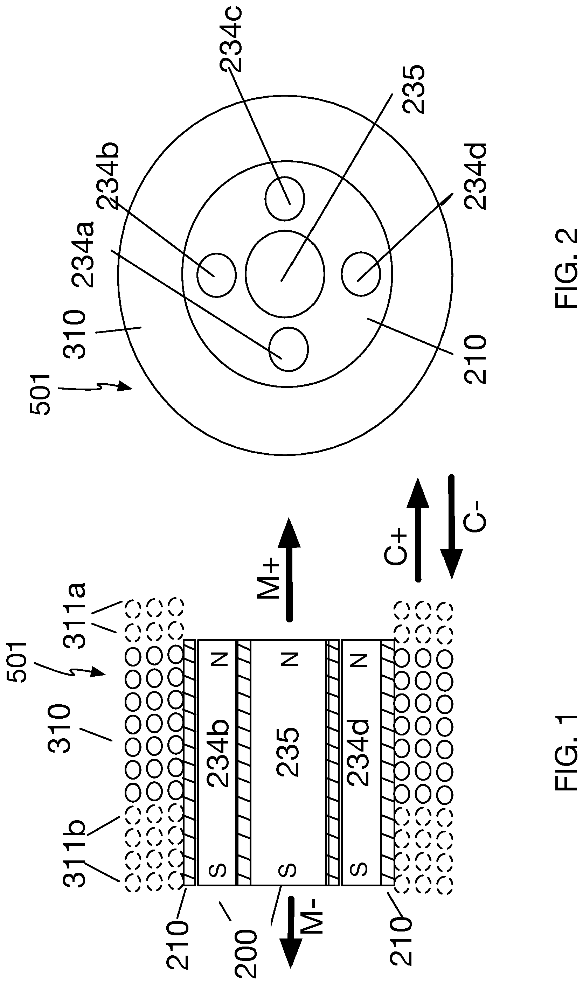

FIGs. 1 and 2 are a cross section and front view of a flux module **501.** A single coil **310** is provided in proximity to a magnet assembly **200**. The coil is wound around the magnet assembly housing **210,** which also serves as a spool for the coil winding. The magnet assembly comprises five cylindrical N52 NIB permanent magnets, which are arranged symmetrically with smaller magnets **235a, 235b, 235c,** and **235d** oriented concentric to a center magnet **234** which has a larger diameter than the other magnets. In one example, the center magnet has a 1.27 cm (0.5 inch) diameter and a length of 3.175 cm (1.25 inches), and the outer magnets have a 0.635 cm (0.25 inch) diameter and a length of 3.175 cm (1.25 inches).

The single coil **310** is wound symmetrically about an axis parallel to the longitudinal axis of the magnet assembly, and extends around a substantial length of the magnet assembly. In other examples, the coil orientation may be asymmetric to the magnet assembly, the coil may be the same width as, or shorter than, the magnet assembly, or overhang the magnet assembly in one or both directions as indicated by coil sections **311a** and **311b.** The coil width to diameter aspect ratio may be substantially more or less than shown.

The permanent magnet assembly provides a permanent magnetic field **M+** in the direction of tissue (not shown). In this specification, **M+** is designated as being directed from the North pole of the magnet assembly. In some configurations, the coil leads (not shown) are introduced at the South pole end of the device. In other configurations, the coil leads are introduced in a manner that does not obstruct either pole region of the magnet assembly, and the device may be used with either the North polarity **M+,** or the South polarity **M-,** field lines directed toward tissue. The latter configuration permits the spaced apart placement of two or more devices in various opposing or attracting polarity orientations as discussed below.

In one example, the coil has approximately 200 turns of 26 gauge insulated wire. In other examples, the number of turns may be less, or substantially higher, and various wire size may be used. This example coil configuration appears to be reasonably "tuned" for various magnet assembly configurations, but is likely to be optimized with further modeling and empirical testing. At least two factors appear to influence coil design. First, there are traditional tradeoffs in all coil-based devices between coil intensity from more coil turns and/or higher coil current, versus the duty cycle and heat buildup in the coil due to wire resistance. Second, the combination of magnet and coils creates an antenna which can be "tuned" to reduce reflected signal and thereby increasing the transmission of coil signal. The reflected signal from inefficient tuning generates heat in the coil control, and increases the energy demand of the device.

The flux module acts as an antenna to transmit the coil electromagnetic field. In order to reduce feedback and increase transmission, it is desirable to "tune" the system as described in references [1, 2]. When the assembly is tuned, less power is wasted on feedback to the controller, and the coil may be energized with less current. In prototype devices, tuning reduced the current from a 12 volt power supply from about 1 amp to less than 0.3 amps, and eliminated heat buildup in the controller. A coil of approximately 200 turns appears to perform well with many N52 NIB permanent magnet assemblies, but is likely to be further optimized.

Since antenna signals can be focused or directed with parabolic or other shaped reflectors, it should be possible to provide rear and/or peripheral shielding with various materials to improve the electromagnetic field transmission. In one example, shielding or reflector elements may be configured as a parabolic antenna positioned behind or around the flux module.

Testing indicates that example assemblies are tuned to provide sufficient intensity and depth of field for good therapeutic results for chronic conditions without cooling of either the coil or the control. Further clinical testing may suggest a need for greater coil intensities to address various conditions, and coil or cooling can be provided with active cooling or static cooling, such as cooling rods.

From one perspective, devices appear to work as an antenna which delivers the coil magnetic field flux substantially deeper into tissue than can be achieved with the coil only.

From another perspective, as suggested by the observed oscillation of small permanent magnets suspended along return magnetic field lines of force, the device appears to "ripple" the permanent magnetic field lines by the coil alternately partially repelling the magnet field lines in the direction of **C1** when coil and magnet polarity is matched, and partially attracting the magnet field lines in the direction of **C2** when the coil and magnet polarity is opposed. This perspective suggests that the coil would be effective with a sequence of only positive or negative current pulses.

It is not practical in medium intensity PEMF devices to provide a large enough coil or enough current to produce a constant magnetic field to match the strength of a strong permanent magnet field. Likewise, it is not practical to create pulses of that strength without accumulating energy to discharge into stronger pulses. Impulse devices use capacitors to provide short pulses that can match or exceed the permanent magnet field, but practical devices cannot be recharged to peak intensity at a rate much greater than a few pulses per second.

Various embodiments of flux modules deliver an increased magnetic flux deeper into tissue that prior art medium intensity PEMF devices. Embodiments have been successfully tested at 900 pulses per second without the need for supplemental coil or controller heat removal, and higher frequencies are likely to be feasible with additional optimization such as faster pulse rise times, shorter pulses, or heat removal.

High intensity impulse PEMF devices can be adapted to incorporate permanent magnet assemblies in order to deliver greater magnetic field flux at higher pulse frequencies (and therefore shorter treatment session times) than can be provided by coils alone.

To date, prototype devices have used the strong N52 NIB magnets, approximately 200 turn coils, and a coil current that can be applied to achieve relatively long pulse duration and relatively high pulse frequency without producing significant heat build-up. Prototype devices have shown unexpected beneficial results for a number of chronic conditions, so development has been directed to improving pulse control, reducing pulse rise times, and designing portable devices. Future research will test various other magnet and coil sizes and configurations, conduct finite element analysis and otherwise characterize and optimize design and control parameters. It may be also be desirable, in some cases, to use stronger permanent magnets as they become available, or weaker permanent magnets.

### Example configurations

In other examples, one or more magnets may be used in the magnet assembly, the magnets may have the same dimensions and strengths, different dimensions, different strengths, and symmetric or asymmetric configurations relative to circular or other layouts. In other examples, the magnet assembly comprises one or more permanent magnet of various shapes and sizes in symmetric or asymmetric alignment, and similar or various magnetic strengths. These examples describe various sizes and orientations of cylindrical or ring magnets. Other magnet shapes, such as bar magnets may be used to achieve different magnetic field characteristics. Magnets within a magnet assembly typically have the same polarity alignment. Other polarity alignments could be used to modify magnetic field profiles.

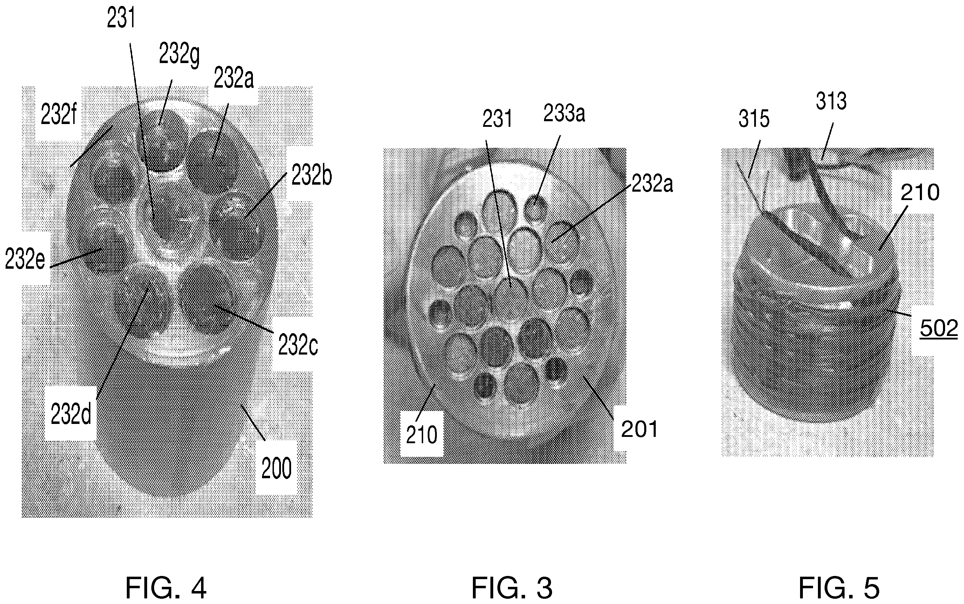

FIG. 3 is a front view of a magnet housing **210** with a central 1.27 cm (0.5 inch) diameter cylindrical magnet **231,** twelve other 1.27 cm (0.5 inch) diameter cylindrical magnets **232;** and six 0.635 cm (0.25 inch) diameter cylindrical magnets **233.**

FIG. 4 is a front perspective view of a magnet assembly **200** with a central 1.27 cm (0.5 inch) diameter cylindrical magnet **231** and six other 1.27 cm (0.5 inch) diameter cylindrical magnets **232a-232g.**

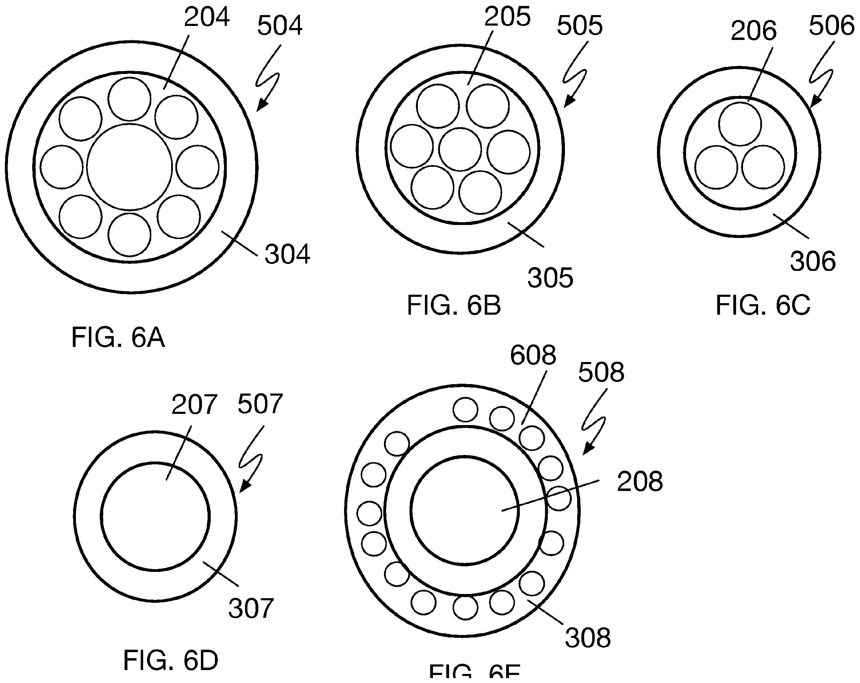

FIG. 6A is a cross section view of an example flux module **504** with a coil assembly **304** and a magnet assembly **204** having a central magnet that is larger than eight surrounding magnets. An example magnet module with a 0.635 cm (0.25 inch) central magnet and 0.3175 cm (0.125 inch) smaller magnets provides good magnetic field depth with a more focussed treatment area.

FIG. 6B is a cross section view of an example flux module **505** with a coil assembly **305** and a magnet assembly **205** with a central magnet surrounded by six magnets of the same size.

FIG. 6C is a cross section view of an example flux module **506** with a coil assembly **306** and a magnet assembly **206** with three magnets of the same size.

FIG. 6D is a cross section view of an example flux module **507** with a coil assembly **307** and a magnet assembly **207** having a single central magnet.

FIG. 6E is a cross section view of an example flux module **508** with a coil assembly **308,** a first magnet assembly **208,** and a second magnet assembly **608.** In this example, the first magnet assembly has a single central magnet, and a second magnet assembly **608** is configured over the coil assembly with a plurality of asymmetrically-arranged smaller magnets. In contrast to the focus provided by the latter example in FIG. 6A, this type of arrangement expands the area of the magnetic field.

In other examples, the magnet assembly comprises a central cylindrical ring magnet oriented within the hole of a ring magnet.

A magnetic strength calculator [REF 3] demonstrates the effect of magnet shape and dimensions on peak intensity and intensity at distance from the head. Without being limited by theory, applicants suggest that these calculated differences are due, in part, to the relative number of "shortcut" magnetic field lines of force between the poles. This suggests that magnetic field intensity at a distance might be increased with various arrangements of multiple smaller magnets rather than a single larger diameter magnet. The use of smaller magnets also reduces device weight and cost.

FIG. 5 is an example of a flux module **502** with first coil lead **313** and second coil lead **315.** Table 1 shows measured magnetic field strengths versus x and y position for the flux module **502.** The magnetic field strength decreases approximately with the square of the distance from the magnet.

### Method of Manufacture

FIG. 9 is a top perspective view of an example aluminum magnet support **210** with a plurality of magnet cavities **212** and a coil retention rim **214.** In other examples, the support is produced by urethane casting. One or more coil is wound around the spool housing and magnets are secured in the cavities. Leads are secured to the coil(s) and the magnet and coil assembly is encapsulated in a fast-curing urethane pour mold with a handle portion to facilitate gripping the flux module. The coil leads are connected or soldered to a coil controller circuit board secured in a housing. The housing supports a lighted on/off switch and a power supply adapter port. In portable devices, the magnet and coil assembly, coil controller circuit board, and battery or capacitor power can be provided in a single housing.

### Coil control

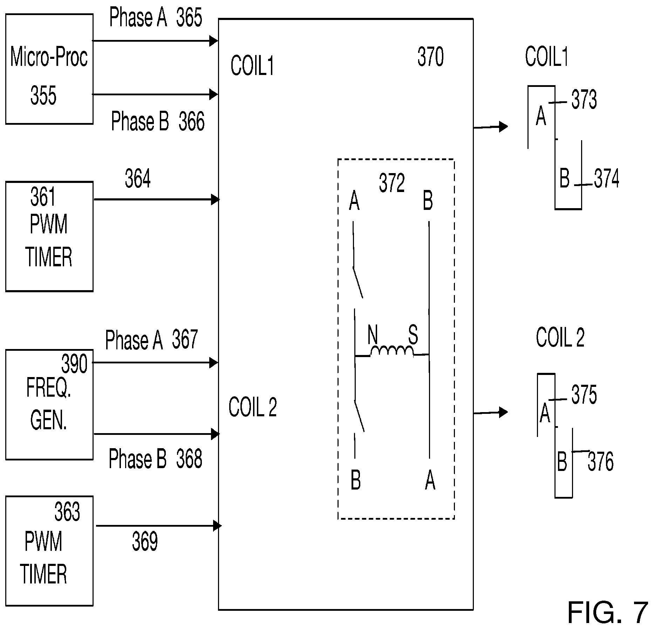

FIG. 7 is an example pulse control schematic. A microprocessor **355** and a pulse width modulation timer **361** provide a first coil signal to a phase controller **370.** The phase controller has an H-bridge **372** that creates positive current pulses **373** and negative current pulses **374.** In various

**Table 1- Field Strength (mT)-1**

| | Distance from face (cm) | | | | | | | |
|---|---|---|---|---|---|---|---|---|
| | **1.0** | **2.0** | **3.0** | **4.0** | **5.0** | **6.0** | **7.0** | **8.0** |
| **Distance from Axis (cm)** | | | | | | | | |
| **3.0** | 49 | 20 | 13 | 11 | 8 | 6 | 5 | 3 |
| **2.0** | 81 | 49 | 26 | 17 | 11 | 7 | 5 | 4 |
| **1.0** | 135 | 65 | 33 | 22 | 13 | 9 | 6 | 4 |
| **0.0** | 155 | 76 | 46 | 23 | 14 | 9 | 6 | 4 |
| **1.0** | 147 | 67 | 37 | 22 | 14 | 8 | 6 | 4 |
| **2.0** | 96 | 50 | 30 | 18 | 13 | 7 | 5 | 4 |
| **3.0** | 41 | 28 | 17 | 14 | 9 | 6 | 4 | 3 |

examples, a second target frequency coil may be driven by a constant frequency or with a variable frequency such as range of audio frequencies. FIG. 7 shows a second coil signal created by a frequency generator **390** and a pulse width modulation timer **361.** A phase controller generates positive current pulses **375** and negative current pulses **376.**

Controls may be provided to select pre-programmed pulse protocols, or to select one or more of pulse intensity, pulse frequency, pulse duration, pulse pattern, or session timing. Prior art has suggested that organisms may adapt to constant frequencies, and that it may be desirable to randomly or otherwise vary the coil frequency and/or coil intensity. For reasons described below, prototype devices were simplified.

### Device performance

Measurements of induced voltage from flux modules are substantially greater than the induced voltage from the coil only. In one example, the difference ranged from about 40% at close range to about 25% at distances of a few centimeters.

Over 30 prototype Permaflux devices have been built with various N52 NIB magnet sizes and configurations, and approximately 200 turn coils with 0.3 amp pulse width modified current at 50 Hz with 9 positive and 9 negative current fast-rise pulses per cycle. The device is typically held against or near the skin or head for two minutes per site. Significant beneficial results have been reported for both in-clinic and at-home device use for a number of chronic conditions including pain and inflammation.

Devices used in two minute sessions appear to provide more effective symptom and/or substantive relief than 20-30 minute TMS treatments for a variety of chronic conditions. These results suggest the importance of an overall "dosage" parameter that incorporates the number of pulse events, the intensity of the events, and the effective reach of those events relative to a target region in the body or brain.

### Applicators and portable devices

In another embodiment, a plurality of flux modules are provided in order to induce voltages over a larger area or volume. As examples, a plurality of flux modules may be affixed to a helmet, headband, belt, vest, or sling support; or provided in a paddle, mat, or mattress housing to deliver flux over a larger area. While magnets having a length to diameter aspect ratio of 2.5 to 5 or more appear to be desirable to improve magnetic field intensity at increased distances from the flux module, larger applicators can employ lower aspect ratio magnets.

The AC powered examples described above have a coil control circuit board that is small enough to be incorporated in a hand-held portable device. In one example, three lithium ion rechargeable batteries, can provide a total voltage of over 11 amps with a battery life that can support many treatment sessions before recharge.

Pods are portable flux modules incorporating smaller power supplies that can be quickly recharged with direct or induction recharged for a few treatments.

### Remote therapy

Prototype devices were designed, at clinic request, for simplicity of operation, with only an on/off switch. Therapy sessions were timed, and the device could be either held against the subject for maximum intensity, or could be spaced away from the subject by up to a centimeter to reduce intensity by about half.

There are several advantages to this approach relative to more complex programming or control options, including consistency of operation, ease of administration or supervision, and lower patient intimidation.

In one example of a home use treatment protocol for a particular ailment, a medical provider can specify device location, device contact or spacing, and treatment session times and frequency. Since the device price is very low compared to clinical devices, the device can be sold, rented, or borrowed for home use; and home use sessions can be monitored by medical staff by a simple video link such as ZOOM^{™} or FACETIME^{™}.

By reducing the need for study participants to travel to a supervised test site, video monitoring can also dramatically reduce administration costs and increase feasible participant sample size for controlled studies of device effectiveness for various ailments.

In other examples, the device may include a communications link which permits remote programming of pulse control parameters and session timing.

### Target frequencies

Operating devices at 900 pulses per second appears to be particularly effective at addressing pain and chronic inflammation, and in strengthening immune response. Other higher or lower pulse rates are also likely to be effective.

In other examples, a second coil is provided, and is operated at a higher targeted frequency than the first coil. In one example, the second coil can be operated a constant frequency in the range of 2000-5000 Hz. Literature suggests that frequencies as high as 1.6 megaHz may be effective. A dual coil device provides two base frequencies as well as sums and differences of the base frequency harmonics. In other examples, the second coil can be operated a variable frequencies. Limited *in vitro* testing suggests the dual coil device operating at a specific target frequency may be effective in killing or weakening pathogens.

### Dual heads with attractive or repulsive orientations

Despite the significant drop in field intensity with distance from the head, the testing of single devices has shown effectiveness for a wide range of ailments.

In some cases, it may be desirable to provide a more intense and focussed magnetic field over a longer distance. The potential effectiveness of using spaced-apart dual flux modules with attracting or opposing heads can be demonstrated with a chamber having iron filings suspended in a viscous liquid. When a first flux module is placed on opposite sides of a chamber several centimeters (inches) from a second flux module having opposite polarity, a horizontal column of filings is suspended between the modules- indicating a strong and near-uniform magnetic field. This type of dual device placement can provide a strong and focused field across a head, torso, leg, or arm.

In other cases, two or more same-polarity devices may be positioned to create repulsive interference regions. Various devices can be positioned to generate a desired magnetic field interference boundary location and shape; and to move that boundary back and forth relative to a desired region of interest.

These types of opposed or attractive polarity configurations may also serve as antennae when a coil is provided with at least one of the magnet assemblies.

### Impulse Devices

The flux module approach shifts the intensity versus distance curve for low to medium intensity PEMF devices toward greater intensity at a given distance from the coil. Since these devices can operate at any pulse frequency, the primary benefit is in improving effective magnetic flux at distance from head.

In some examples, it may be desirable to provide flux modules with much higher coil current than can be supplied without a voltage multiplier. Prior art impulse devices can only provide peak intensity at low pulse rates. For example, an impulse PEMF vendor reported 2020 data showing a peak intensity of 0.14 T (1400 Gauss) at 0.54 pulses per second; but dropping to 0.035 T (350 Gauss) at 6 pulses per second and to 0.022 T (220 Gauss) at 10 pulses per second.

The flux module approach of combining a magnet assembly with high intensity coils in impulse PEMF devices can provide greater intensity, more frequent pulses, or both.

For a given intensity at or below peak, a flux module approach could enable a higher pulse rate, so that treatment times could be reduced by a factor of 2-3X or more. In other cases, such as pelvic muscle contraction, TMS , or migraine therapy, where slow pulse rates may be preferred, a flux module approach -particularly in a dual head opposed polarity configuration- could substantially reduce the required coil size or coil current to match or exceed coil-only performance.

### Ailments

Based on initial clinic use, home use, and device measurements, the embodiments described herein appear to be more effective than prior art PEMF devices in addressing a variety of acute and chronic ailments. The devices appear to be particularly effective in reducing inflammation so that the body's immune system can address those ailments. Many of the applications of PEMF are summarized in US Patent 8376925 to Dennis et al.

While these embodiments have been described with emphasis on the embodiments, it should be understood that within the scope of the appended claims, the embodiments might be practiced other than as specifically described herein. In particular various configurations of coil assemblies, magnet assemblies, and pulse intensities, pulse duration, pulse rise times, treatment protocols, and device placement may be used. The invention is defined by the appended claims.

## Claims

1. A pulsed electromagnetic field device configured to apply a fluctuating electromagnetic field to a subject organism, the pulsed electromagnetic field device comprising
a flux module (500, 501, 502, 504, 505, 506, 507, 508) comprising
a permanent magnet assembly (204, 206, 207, 208) comprising
permanent magnet housing (210),
a plurality of parallel cylindrical permanent magnets (232, 232a, 232b, 232c, 232d, 232e, 232f, 232g, 233, 234, 235a, 235b, 235c, 235d) supported in the permanent magnet housing, each magnet having a first end with North polarity and a second end with South polarity, and a longitudinal axis between the first end and the second end
such that the plurality of permanent magnets project a permanent magnetic field, and
a coil assembly (304, 310) comprising a first coil wound about the permanent magnet assembly;
a power supply configured to provide a voltage in the range of 6 to 24 volts; and
a first coil controller configured to deliver a sequence of pulse width modulated current pulses to the first coil, wherein the first coil controller is further configured to provide a sequence of positive and negative current pulses
to the first coil, such that each pulse has a rise time less than 5 microseconds, such that
when the first coil is energized with a positive current direction, the coil generates an electromagnetic field with the same polarity as the magnet assembly, thereby enhancing the permanent magnetic field, and when the first coil is energized with a negative current direction, the coil generates an electromagnetic field with the opposite polarity as the magnet assembly, thereby partially retracting the permanent magnetic field.

2. The pulsed electromagnetic field device of claim 1 wherein the first coil is wound around the permanent magnet assembly with respect to a longitudinal axis of the permanent magnet assembly, and configured perpendicular to the longitudinal axes of the cylindrical magnets.

3. The pulsed electromagnetic field device of claim 1 wherein the first coil has between 100 and 500 turns.

4. The pulsed electromagnetic field device of claim 1 wherein the power supply comprises line voltage, at least one battery, or at least one capacitor.

5. The pulsed electromagnetic field device of claim 1 wherein the first coil controller is configured to deliver a sequence of current pulses in the range of 1 to 1000 pulses per second.

6. The pulsed electromagnetic field device of claim 1, further comprising a voltage multiplier, wherein the voltage multiplier is configured to deliver a series of current pulses sufficient to produce a peak magnetic field intensity in the range of 0.1 to 2.5 T (1,000 to 25,000 gauss).

7. The pulsed electromagnetic field device of claim 1 wherein the coil assembly further comprises a second coil wound about the permanent magnet assembly, and a second coil controller.

8. The pulsed electromagnetic field device of claim 1 further comprising a focus element positioned around at least a portion of the coil assembly.

9. The pulsed electromagnetic field device of claim 1 wherein each cylindrical permanent magnet has a length of at least 2.54 cm (one inch); the first coil has 150-250 turns; and the first coil controller is configured to deliver a sequence of positive and negative current pulses, from an input voltage of 6-14 volts, at a rate of 800 to 1000 pulses per second

## Patentansprüche

1. Gepulstes elektromagnetisches Feldgerät, das dazu konfiguriert ist, ein schwankendes elektromagnetisches Feld an einen Subjektorganismus anzulegen, wobei das gepulste elektromagnetische Feldgerät umfasst:
ein Flussmodul (500, 501, 502, 504, 505, 506, 507, 508), umfassend:
eine Permanentmagnetanordnung (204, 206, 207, 208), umfassend ein Permanentmagnetgehäuse (210),
eine Vielzahl paralleler zylindrischer Permanentmagnete (232, 232a, 232b, 232c, 232d, 232e, 232f, 232g, 233, 234, 235a, 235b, 235c, 235d), die im Permanentmagnetgehäuse gehalten sind, wobei jeder Magnet ein erstes Ende mit Nordpolarität und ein zweites Ende mit Südpolarität, und eine Längsachse zwischen dem ersten Ende und dem zweiten Ende aufweist, derart, dass die Vielzahl der Permanentmagnete ein permanentes Magnetfeld erzeugt, und
eine Spulenanordnung (304, 310), umfassend eine erste Spule, die um die Permanentmagnetanordnung gewickelt ist;
eine Stromversorgung, die dazu konfiguriert ist, eine Spannung im Bereich von 6 bis 24 Volt bereitzustellen; und
einen ersten Spulencontroller, der dazu konfiguriert ist, eine Folge pulsweitenmodulierter Stromimpulse an die erste Spule zu liefern, wobei der erste Spulencontroller ferner dazu konfiguriert ist, der ersten Spule eine Folge positiver und negativer Stromimpulse zuzuführen, wobei jeder Impuls eine Anstiegszeit von weniger als 5 Mikrosekunden aufweist, wobei die erste Spule, wenn sie mit einer positiven Stromrichtung erregt wird, ein elektromagnetisches Feld mit derselben Polarität wie die Magnetanordnung erzeugt und dadurch das permanente Magnetfeld verstärkt, und wobei die erste Spule, wenn sie mit einer negativen Stromrichtung erregt wird, ein elektromagnetisches Feld mit der entgegengesetzten Polarität wie die Magnetanordnung erzeugt und dadurch das permanente Magnetfeld teilweise zurückzieht.

2. Gepulstes elektromagnetisches Feldgerät nach Anspruch 1, wobei die erste Spule in Bezug auf eine Längsachse der Permanentmagnetanordnung um die Permanentmagnetanordnung gewickelt und senkrecht zu den Längsachsen der zylindrischen Magnete ausgerichtet ist.

3. Gepulstes elektromagnetisches Feldgerät nach Anspruch 1, wobei die erste Spule zwischen 100 und 500 Windungen aufweist.

4. Gepulstes elektromagnetisches Feldgerät nach Anspruch 1, wobei die Stromversorgung Netzspannung, mindestens eine Batterie oder mindestens einen Kondensator umfasst.

5. Gepulstes elektromagnetisches Feldgerät nach Anspruch 1, wobei der erste Spulencontroller dazu konfiguriert ist, eine Folge von Stromimpulsen im Bereich von 1 bis 1000 Impulsen pro Sekunde zu liefern.

6. Gepulstes elektromagnetisches Feldgerät nach Anspruch 1, ferner umfassend einen Spannungsvervielfacher, wobei der Spannungsvervielfacher dazu konfiguriert ist, eine Reihe von Stromimpulsen zu liefern, die ausreicht, um eine Spitzenmagnetfeldintensität im Bereich von 0,1 bis 2,5 T (1.000 bis 25.000 Gauss) zu erzeugen.

7. Gepulstes elektromagnetisches Feldgerät nach Anspruch 1, wobei die Spulenanordnung ferner eine zweite Spule umfasst, die um die Permanentmagnetanordnung gewickelt ist, und einen zweiten Spulencontroller.

8. Gepulstes elektromagnetisches Feldgerät nach Anspruch 1, ferner umfassend ein Fokussierelement, das um mindestens einen Teil der Spulenanordnung positioniert ist.

9. Gepulstes elektromagnetisches Feldgerät nach Anspruch 1, wobei jeder zylindrische Permanentmagnet eine Länge von mindestens 2,54 cm (ein Zoll) aufweist;
die erste Spule 150 - 250 Windungen aufweist; und
der erste Spulencontroller dazu konfiguriert ist, aus einer Eingangsspannung von 6 bis 14 Volt eine Folge positiver und negativer Stromimpulse mit einer Rate von 800 bis 1000 Impulsen pro Sekunde zu liefern.

## Revendications

1. Dispositif à champ électromagnétique pulsé configuré pour appliquer un champ électromagnétique fluctuant à un organisme sujet, le dispositif à champ électromagnétique pulsé comprenant :
un module de flux (500, 501, 502, 504, 505, 506, 507, 508) comprenant :
un ensemble d'aimants permanents (204, 206, 207, 208) comprenant un boîtier d'aimants permanents (210),
une pluralité d'aimants permanents cylindriques parallèles (232, 232a, 232b, 232c, 232d, 232e, 232f, 232g, 233, 234, 235a, 235b, 235c, 235d) supportés dans le boîtier d'aimants permanents, chaque aimant ayant une première extrémité de polarité Nord, une seconde extrémité de polarité Sud, et un axe longitudinal entre la première extrémité et la seconde extrémité,
de sorte que la pluralité d'aimants permanents projette un champ magnétique permanent, et
un ensemble de bobines (304, 310) comprenant une première bobine enroulée autour de l'ensemble d'aimants permanents ;
une alimentation électrique configurée pour fournir une tension dans la plage de 6 à 24 volts ; et
un premier contrôleur de bobine configuré pour délivrer à la première bobine une séquence d'impulsions de courant modulées en largeur d'impulsion, dans lequel le premier contrôleur de bobine ést en outre configuré pour fournir à la première bobine une séquence d'impulsions de courant positives et négatives, de sorte que chaque impulsion présente un temps de montée inférieur à 5 microsecondes, de sorte que, lorsque la première bobine est excitée avec un sens de courant positif, la bobine génère un champ électromagnétique ayant la même polarité que l'ensemble d'aimants permanents, renforçant ainsi le champ magnétique permanent, et que, lorsque la première bobine est excitée avec un sens de courant négatif, la bobine génère un champ électromagnétique ayant une polarité opposée à celle de l'ensemble d'aimants permanents, rétractant ainsi partiellement le champ magnétique permanent.

2. Dispositif à champ électromagnétique pulsé selon la revendication 1, dans lequel la première bobine est enroulée autour de l'ensemble d'aimants permanents par rapport à un axe longitudinal de l'ensemble d'aimants permanents, et est configurée perpendiculairement aux axes longitudinaux des aimants cylindriques.

3. Dispositif à champ électromagnétique pulsé selon la revendication 1, dans lequel la première bobine a entre 100 et 500 spires.

4. Dispositif à champ électromagnétique pulsé selon la revendication 1, dans lequel l'alimentation électrique comprend une tension de ligne, au moins une batterie ou au moins un condensateur.

5. Dispositif à champ électromagnétique pulsé selon la revendication 1, dans lequel le premier contrôleur de bobine est configuré pour délivrer une séquence d'impulsions de courant dans la plage de 1 à 1000 impulsions par seconde.

6. Dispositif à champ électromagnétique pulsé selon la revendication 1, comprenant en outre un multiplicateur de tension, dans lequel le multiplicateur de tension est configuré pour délivrer une série d'impulsions de courant suffisante pour produire une intensité de crête du champ magnétique dans la plage de 0,1 à 2,5 T (1 000 à 25 000 gauss).

7. Dispositif à champ électromagnétique pulsé selon la revendication 1, dans lequel l'ensemble de bobines comprend en outre une seconde bobine enroulée autour de l'ensemble d'aimants permanents, et un second contrôleur de bobine.

8. Dispositif à champ électromagnétique pulsé selon la revendication 1, comprenant en outre un élément de focalisation disposé autour d'au moins une partie de l'ensemble de bobines.

9. Dispositif à champ électromagnétique pulsé selon la revendication 1, dans lequel chaque aimant permanent cylindrique a une longueur d'au moins 2,54 cm (un pouce) ; la première bobine comporte 150 - 250 spires ; et le premier contrôleur de bobine est configuré pour délivrer une séquence d'impulsions de courant positives et négatives, à partir d'une tension d'entrée de 6 à 14 volts, à une cadence de 800 à 1000 impulsions par seconde.
